# FASCICULE DE BREVET EUROPEEN

(11) **EP 3 721 810 B1**
(45) Date de publication et mention de la délivrance du brevet: **12.07.2023**
(21) Numéro de dépôt: 19168289.7
(22) Date de dépôt: 09.04.2019
(51) Int. Cl.: A61B 10/00

(54) **COLLECTEUR URINAIRE POUR HOMME ET KIT DE PRÉLÈVEMENT URINAIRE COMPRENANT CE COLLECTEUR**
URINSAMMELBEHÄLTER FÜR MÄNNER UND URIN-ENTNAHMEKIT, DAS EINEN SOLCHEN SAMMELBEHÄLTER UMFASST
URINE COLLECTOR FOR MEN AND URINE SAMPLING KIT COMPRISING THIS COLLECTOR

(43) Date de publication de la demande: 14.10.2020
(73) Titulaire: Rabhi, Abdelhamid, 91270 Vigneux-sur-Seine (FR)
(72) Inventeur: Rabhi, Abdelhamid, 91270 Vigneux-sur-Seine (FR)
(74) Mandataire: Koudine, Andreï

(56) Documents cités:
- WO-A1-2013/082397
- US-A- 3 161 891
- US-A- 4 202 058
- US-A- 4 213 598
- US-A- 5 622 183
- US-A1- 2006 064 034
- US-A1- 2007 270 716

## Description

La présente invention concerne, de manière générale, le domaine des équipements sanitaires portables comme ceux décrits dans US5622183A qui sont agencés pour l'homme et adaptés à recevoir une urine humaine. Le terme « homme » est utilisé ici au sens « être humain » et, de ce fait, signifie soit un être humain de sexe masculin communément appelé « homme », soit un être humain de sexe féminin communément appelé « femme ».

Plus particulièrement, selon un premier de ses aspects, l'invention concerne un collecteur urinaire pour homme comportant un récipient ouvert vers l'extérieur et présentant un volume intérieur. En outre, le collecteur urinaire comporte un réceptacle anatomique présentant un bord extérieur destiné à être orienté vers un corps humain. Ce bord extérieur est apte à coopérer avec le corps humain au niveau du méat urétral. Le réceptacle anatomique comprend un orifice de sortie disposé à l'opposé du bord extérieur. Le récipient est adapté à être connecté de manière amovible au réceptacle anatomique. Le récipient est en outre apte à coopérer avec l'orifice de sortie du réceptacle anatomique. Le réceptacle anatomique est adapté à capturer une urine éjectée du méat urétral lors de la miction et à canaliser cette urine, sous l'effet de la pesanteur, via l'orifice de sortie, vers le récipient. Le volume intérieur du récipient est adapté à stocker l'urine issue du réceptacle anatomique.

Un tel collecteur urinaire est connu et décrit, par exemple, dans le brevet chinois CN2261239Y. Ce collecteur urinaire connu peut être utilisé dans un contexte hospitalier pour capturer l'urine éjectée par un patient, par exemple, allongé et/ou au moins partiellement immobile. Ce dernier peut présenter un état de santé qui nécessite une analyse médicale (par exemple, expresse) de son urine. Cependant, l'urine stockée dans le collecteur urinaire connu présente un taux d'impuretés élevé. Ainsi, son utilisation à des fins d'analyses médicales est a priori exclue ce qui est insatisfaisant.

La présente invention, qui s'appuie sur cette observation originale, a principalement pour but de proposer un collecteur urinaire visant au moins à réduire une limitation précédemment évoquée. A cette fin, le collecteur urinaire est stérile.

Cet agencement permet d'éviter une contamination hasardeuse de l'urine capturée par le collecteur urinaire selon l'invention par des impuretés. Autrement dit, l'urine issue du collecteur urinaire selon l'invention peut être assimilée à un échantillon urinaire prélevé chez le patient à des fins médicales. De ce fait, cette urine est adapté pour des analyses médicales.

De préférence, au moins le réceptacle anatomique est fabriqué en matériau recyclable.

Cet agencement facilite un traitement des déchets formés par des collecteurs urinaires (après leurs usages), contribue à protéger un environnement et est respectueux des normes du développement durable en vigueur.

De préférence, au moins le réceptacle anatomique est fabriqué en carton.

Cet agencement facilite un traitement des déchets formés par des collecteurs urinaires (après leurs usages), contribue à protéger un environnement et est respectueux des normes du développement durable en vigueur. En plus, cet agencement permet d'obtenir des collecteurs urinaires jetables adaptés à un usage unique. Cette solution vise à contenir des germes et de limiter leurs propagations, notamment dans un milieu hospitalier. De ce fait, cet agencement contribue à renforcer la santé publique. Enfin, le réceptacle anatomique en carton présente un poids limité ce qui permet de faciliter son transport et, in fine, de réduire une empreinte environnementale écologique due aux émissions (notamment celles de CO2) lors du transport de chaque collecteur urinaire.

Le récipient comporte un appendice tubulaire ondulé flexible apte à être déplié vers l'extérieur hors réceptacle anatomique de manière à augmenter le volume intérieur dudit récipient.

Cet agencement élargit des possibilités fonctionnelles du collecteur urinaire selon l'invention et, in fine, le rend plus universel à l'usage. En effet, sa géométrie peut être immédiatement adaptée en fonction d'un volume de la vessie de chaque utilisateur.

De préférence, l'appendice tubulaire ondulé flexible forme une bouche d'accès au volume intérieur du récipient, la bouche d'accès étant apte à coopérer avec l'orifice de sortie du réceptacle anatomique.

Grâce à cet agencement, l'appendice tubulaire ondulé flexible est disposé entre le réceptacle anatomique et le récipient. Ainsi, il est plus facile (et plus intuitive) pour un utilisateur de déplier, si besoin, l'appendice tubulaire ondulé flexible : pour cela il suffit de saisir le récipient et de le tirer hors du réceptacle anatomique. De même, il est possible de plier de manière sélective l'appendice tubulaire ondulé flexible pour assurer une disposition optimale du réceptacle anatomique par rapport au récipient et/ou à la pesanteur pour rendre plus aisé un prélèvement urinaire d'un patient immobile (par exemple, suite à une paralysie d'au moins une partie du corps.

De préférence, le réceptacle anatomique comporte un bec verseur disposé sur le bord extérieur, le bec verseur étant adapté à faciliter une évacuation d'un excédent de l'urine par-dessus bord du réceptacle anatomique.

Cet agacement rend plus aisée une utilisation d'un collecteur urinaire selon l'invention, par exemple, par des personnes à mobilité réduite, et/ou des personnes âgées, et/ou, plus généralement, par toute personne qui ne peut pas contrôler avec précision ses mouvements (ceux des mains, du corps etc.).

De préférence, le réceptacle anatomique est fabriqué en matériau pliable. Dans ces conditions, le bec verseur est formé par pliage du réceptacle anatomique.

Cet agencement contribue à rendre le collecteur urinaire selon l'invention plus compacte. En effet, le réceptacle anatomique pliable présente un volume limité ce qui permet de faciliter son transport et, in fine, de réduire une empreinte environnementale écologique due aux émissions (notamment celles de CO2) lors du transport de chaque collecteur urinaire. De même, cet agencement facilite un traitement des déchets formés par des collecteurs urinaires (après leurs usages), contribue à protéger un environnement et est respectueux des normes du développement durable en vigueur.

De préférence, le collecteur urinaire selon l'invention comporte un filtre d'urine disposé en amont du récipient.

Grâce à cet agencement, des corps étrangers (par exemples, des poiles) ne peuvent pas contaminer l'urine stockée dans le récipient.

De préférence, le réceptacle anatomique comporte un dispositif de connexion adapté à connecter de manière amovible le réceptacle anatomique au récipient. Dans ces conditions, ce dispositif de connexion comporte une première extrémité liée solidairement au réceptacle anatomique et une deuxième extrémité libre, opposée à la première extrémité. Le récipient est adapté à être connecté à la deuxième extrémité libre à l'aide d'un lien amovible.

Grâce à cet agencement, les contraintes produites lors de la fixation du réceptacle anatomique au récipient se concentrent au niveau du dispositif de connexion : il est donc possible de minimiser une épaisseur du réceptacle anatomique de manière à le rendre plus léger, et/ou moins volumineux, et/ou plus aisément pliable.

De préférence, le lien amovible est de nature mécanique, par exemple, à l'aide d'un raccord fileté.

Cet agencement contribue à minimiser des fuites de l'urine entre la deuxième extrémité libre du dispositif de connexion et le récipient.

De manière alternative, il est possible que le lien amovible soit de nature magnétique.

Cette solution rend plus aisée la fixation du récipient à la deuxième extrémité libre du dispositif de connexion.

De préférence, le dispositif de connexion comporte un anneau. Dans ces conditions, l'anneau est disposé autour de l'orifice de sortie.

Cet agencement contribue à rendre plus aisée une fabrication du collecteur urinaire à l'échelle industrielle.

De préférence, le réceptacle anatomique comporte un dispositif antifuite adapté à au moins limiter une infiltration de l'urine à travers une zone de connexion formée entre le réceptacle anatomique et le récipient au niveau de la deuxième extrémité libre de l'anneau.

Cet agencement permet aux utilisateurs de ne pas se salir les mains avec de l'urine lors d'un prélèvement urinaire à l'aide d'un collecteur urinaire selon l'invention. Cette solution vise à contenir des germes et de limiter leurs propagations, notamment dans un milieu hospitalier. De ce fait, cet agencement contribue à renforcer la santé publique et rend le collecteur urinaire selon l'invention plus hygiénique à l'usage.

De préférence, le dispositif antifuite comporte une buse d'injection assise sur l'orifice de sortie du réceptacle anatomique. Dans ces conditions, la buse d'injection est allongée le long de l'anneau jusqu'au-delà de la deuxième extrémité libre de l'anneau. La buse d'injection comporte une tête de buse apte à orienter l'urine issue du réceptacle anatomique dans le récipient, hors zone de connexion formée entre le réceptacle anatomique et le récipient au niveau de la deuxième extrémité libre de l'anneau.

Cet agencement permet aux utilisateurs de ne pas se salir les mains avec de l'urine lors d'un prélèvement urinaire à l'aide d'un collecteur urinaire selon l'invention. Cette solution vise à contenir des germes et de limiter leurs propagations, notamment dans un milieu hospitalier. De ce fait, cet agencement contribue à renforcer la santé publique et rend le collecteur urinaire selon l'invention plus hygiénique à l'usage.

De préférence, la buse d'injection comporte un canal interne apte à canaliser l'urine, en aval de l'orifice de sortie, sous l'effet de la pesanteur, hors du réceptacle anatomique. Dans ces conditions, ce canal interne est fabriqué en forme d'entonnoir se rétrécissant de l'orifice de sortie du réceptacle anatomique vers la tête de buse.

Cet agencement permet de contrôler avec précision un écoulement de l'urine, sous l'effet de la pesanteur, du réceptacle anatomique vers le récipient, de manière à éviter toute fuite dans la zone de connexion formée entre le réceptacle anatomique et le récipient au niveau de la deuxième extrémité libre de l'anneau.

De préférence, la buse d'injection présente un contour extérieur en forme d'entonnoir se rétrécissant de l'orifice de sortie du réceptacle anatomique vers la tête de buse.

Cet agencement vise à réduire une épaisseur de la buse d'injection et, in fine, de rendre plus léger le collecteur urinaire selon l'invention.

Selon un deuxième de ses aspects, l'invention concerne un kit de prélèvement urinaire pour homme comprenant un collecteur urinaire pour homme comportant un récipient ouvert vers l'extérieur et présentant un volume intérieur. En outre, le collecteur urinaire comporte un réceptacle anatomique présentant un bord extérieur destiné à être orienté vers un corps humain. Ce bord extérieur est apte à coopérer avec le corps humain au niveau du méat urétral. Le réceptacle anatomique comprend un orifice de sortie disposé à l'opposé du bord extérieur. Le récipient est adapté à être connecté de manière amovible au réceptacle anatomique. Le récipient est en outre apte à coopérer avec l'orifice de sortie du réceptacle anatomique. Le réceptacle anatomique est adapté à capturer une urine éjectée du méat urétral lors de la miction et à canaliser cette urine, sous l'effet de la pesanteur, via l'orifice de sortie, vers le récipient. Le volume intérieur du récipient est adapté à stocker l'urine issue du réceptacle anatomique.

Selon l'invention, le collecteur urinaire est stérile. Le kit de prélèvement urinaire comprend un emballage étanche adapté à envelopper le collecteur urinaire. Cet emballage étanche est conditionné sous une atmosphère modifiée adaptée à protéger le collecteur urinaire contre une contamination microbienne.

Ces agencements permettent de protéger le collecteur urinaire contre une contamination microbienne tout au long de son chemin (de l'usine à l'utilisateur).

De préférence, l'atmosphère modifiée comporte au moins un gaz inerte.

Grâce à cet agencement, toute prolifération de germes à l'intérieur du kit de prélèvement urinaire est proscrite.

De préférence, le kit de prélèvement urinaire comporte un bouchon stérile adapté à obturer le récipient.

Grâce à cet agencement, l'utilisateur peut obturer le récipient une fois que ce dernier soit déconnecté du réceptacle anatomique après le prélèvement urinaire. Le bouchon étant stérile, l'urine prélevée est protégée contre une contamination microbienne.

D'autres caractéristiques et avantages de l'invention ressortiront clairement de la description qui en est faite ci-après, à titre indicatif et nullement limitatif, en référence aux dessins annexés.

### Brève description des figures

[Figure 1] représente schématiquement, en vue isométrique simplifiée, un collecteur urinaire (1) selon l'invention adapté à un être humain de sexe masculin, ce collecteur urinaire (1) comportant un récipient (12) connecté (par exemple, par vissage ou clipsage) au réceptacle anatomique (13).
[Figure 2] représente schématiquement, en vue isométrique simplifiée, un collecteur urinaire (1) selon l'invention adapté à un être humain de sexe masculin, ce collecteur urinaire (1) comportant un récipient (12) déconnecté du réceptacle anatomique (13).
[Figure 3] représente schématiquement, en vue isométrique simplifiée, un collecteur urinaire (1) selon l'invention adapté à un être humain de sexe féminin, ce collecteur urinaire (1) comportant un récipient (12) vissé au réceptacle anatomique (13).
[Figure 4] représente schématiquement, en première vue de côté simplifiée, un collecteur urinaire (1) selon l'invention adapté à un être humain de sexe féminin, ce collecteur urinaire (1) comportant un récipient (12) vissé au réceptacle anatomique (13).
[Figure 5] représente schématiquement, en deuxième vue de côté simplifiée, un collecteur urinaire (1) selon l'invention adapté à un être humain de sexe féminin, ce collecteur urinaire (1) comportant un récipient (12) vissé au réceptacle anatomique (13).
[Figure 6] représente schématiquement, en vue de dessus simplifiée, un collecteur urinaire (1) selon l'invention représenté sur les figures 3 à 5 et adapté à un être humain de sexe féminin.
[Figure 7] représente schématiquement un kit de prélèvement urinaire comportant un collecteur urinaire (1) selon l'invention représenté sur les figures 3 à 6 et adapté à un être humain de sexe féminin, ce collecteur urinaire (1) comportant un récipient (12) déconnecté du réceptacle anatomique (13).
[Figure 8] représente schématiquement un kit de prélèvement urinaire comportant un collecteur urinaire (1) selon l'invention muni d'une buse d'injection (15) et adapté à un être humain de sexe féminin, ce collecteur urinaire (1) comportant un récipient (12) connecté (par vissage ou par clipsage) au réceptacle anatomique (13).

### Description détaillée de l'invention

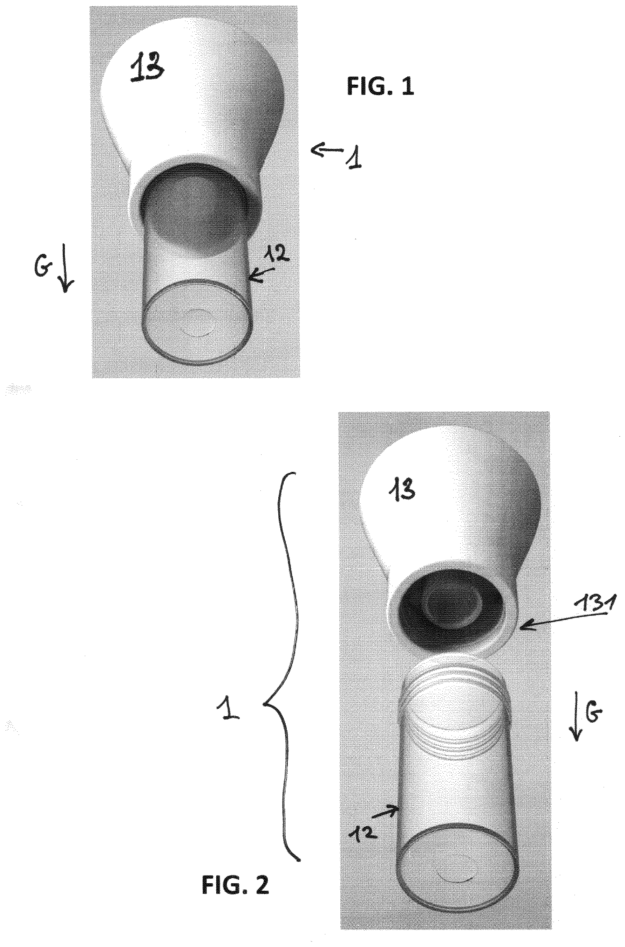

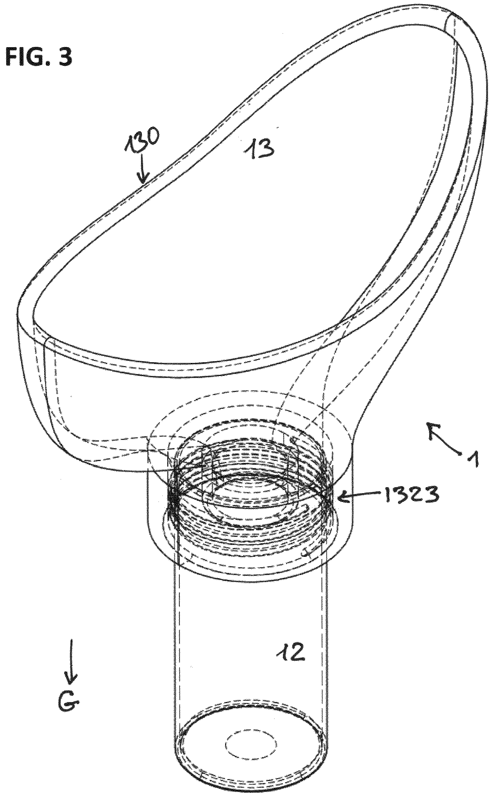

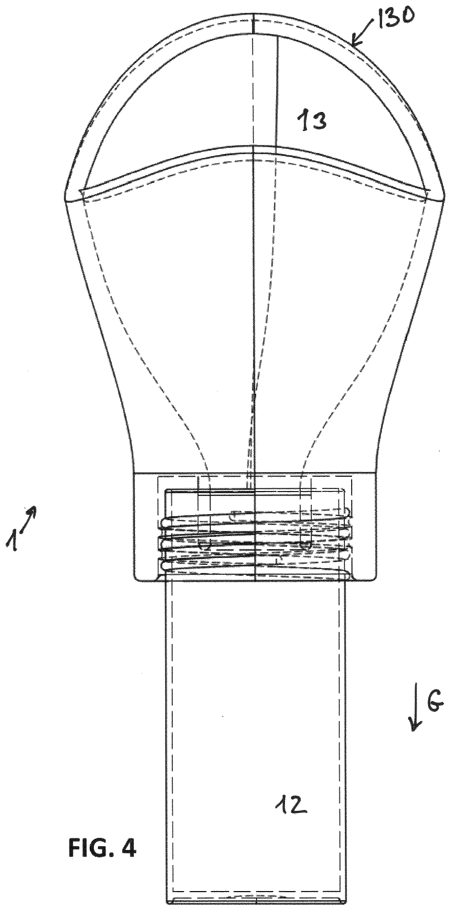

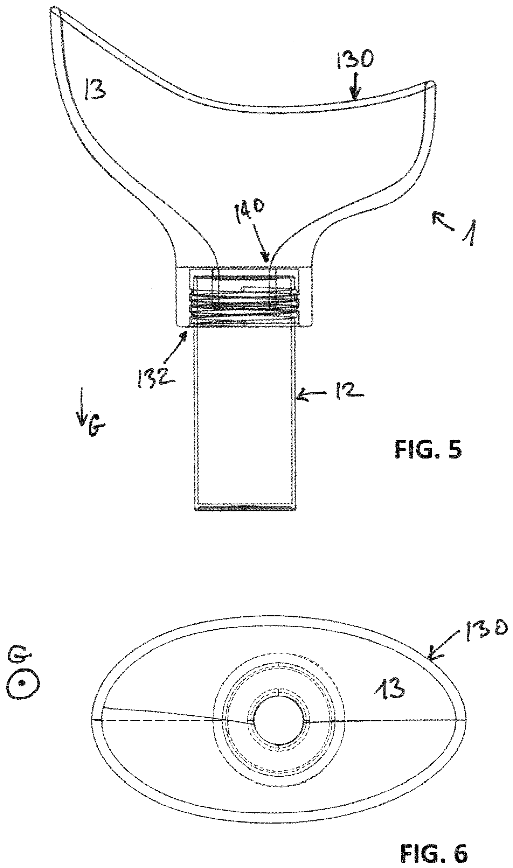

Comme annoncé précédemment et illustré sur les figures 1 à 8, l'invention concerne, selon un premier de ses aspects, un collecteur urinaire (1) pour homme comportant un récipient (12) ouvert vers l'extérieur et présentant un volume intérieur, le collecteur urinaire (1) comportant en outre un réceptacle anatomique (13) présentant un bord extérieur (130) destiné à être orienté vers un corps humain, ce bord extérieur (130) étant apte à coopérer avec le corps humain au niveau du méat urétral, le réceptacle anatomique (13) comprenant un orifice de sortie (131) disposé à l'opposé du bord extérieur (130), le récipient (12) étant adapté à être connecté de manière amovible au réceptacle anatomique (13), le récipient (12) étant en outre apte à coopérer avec l'orifice de sortie (131) du réceptacle anatomique (13), le réceptacle anatomique (13) étant adapté à capturer une urine éjectée du méat urétral lors de la miction et à canaliser cette urine, sous l'effet de la pesanteur (G), via l'orifice de sortie (131), vers le récipient (12), le volume intérieur du récipient (12) étant adapté à stocker l'urine issue du réceptacle anatomique (13).

Selon l'invention, au moins une partie du collecteur urinaire (1) est stérile.

Avantageusement, au moins le réceptacle anatomique (13) peut être fabriqué en carton.

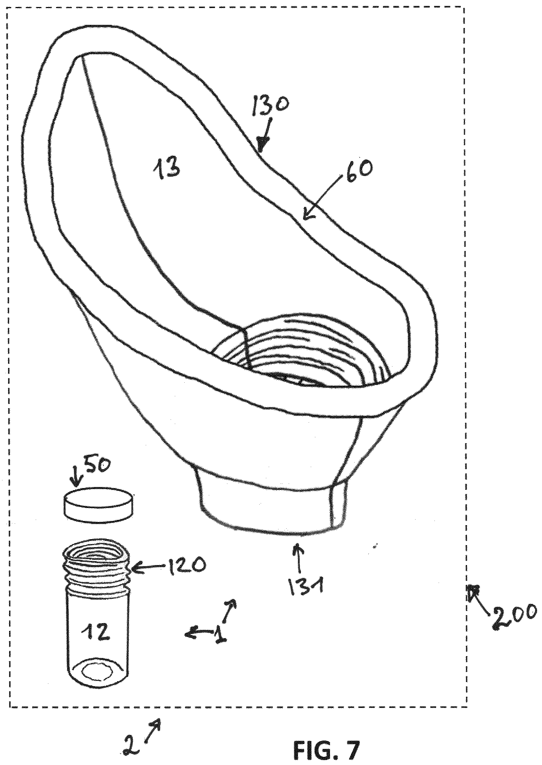

Comme illustré sur la figure 7, le récipient (12) comporte un appendice tubulaire ondulé flexible (120) apte à être déplié vers l'extérieur hors réceptacle anatomique (13) de manière à augmenter le volume intérieur dudit récipient (12).

Cet appendice tubulaire ondulé flexible (120) forme une bouche d'accès au volume intérieur du récipient (12), la bouche d'accès étant apte à coopérer avec l'orifice de sortie (131) du réceptacle anatomique (13).

Comme illustré sur les figures 1-6, 8, le réceptacle anatomique (13) comporte un dispositif de connexion (132) adapté à connecter de manière amovible le réceptacle anatomique (13) au récipient (12). Ce dispositif de connexion (132) comporte une première extrémité (1320) liée solidairement au réceptacle anatomique (13) et une deuxième extrémité libre (1322), opposée à la première extrémité (1320). Le récipient (12) est adapté à être connecté à la deuxième extrémité libre (1322) à l'aide d'un lien amovible.

Le dispositif de connexion (132) comporte un anneau (1323). L'anneau (1323) est disposé autour de l'orifice de sortie (131).

Le réceptacle anatomique (13) comporte un dispositif antifuite adapté à au moins limiter une infiltration de l'urine à travers une zone de connexion formée entre le réceptacle anatomique (13) et le récipient (12) au niveau de la deuxième extrémité libre (1322) de l'anneau (1323).

Le dispositif antifuite comporte une buse d'injection (140) assise sur l'orifice de sortie (131) du réceptacle anatomique (13). Dans ces conditions, la buse d'injection (140) est allongée le long de l'anneau (1323) jusqu'au-delà de la deuxième extrémité libre (1322) de l'anneau (1323). La buse d'injection (140) comporte une tête de buse (15) apte à orienter l'urine issue du réceptacle anatomique dans le récipient (12), hors zone de connexion formée entre le réceptacle anatomique (13) et le récipient (12) au niveau de la deuxième extrémité libre (1322) de l'anneau (1323).

La buse d'injection (140) comporte un canal interne (16) apte à canaliser l'urine, en aval de l'orifice de sortie, sous l'effet de la pesanteur (G), hors du réceptacle anatomique (13). Ce canal interne (16) est fabriqué en forme d'entonnoir se rétrécissant de l'orifice de sortie (131) du réceptacle anatomique (13) vers la tête de buse (15).

Dans une variante de réalisation non illustrée sur les figures, le réceptacle anatomique (13) peut être scellé au niveau du bord extérieur (130) et de l'orifice de sortie (131) par un pellicule étanche (par exemple, en papier ou en film de polymère).

Cet agencement contribue à protéger davantage une paroi interne réceptacle anatomique (13) contre une contamination microbienne.

De préférence, dans cette variante de l'invention, le réceptacle anatomique (13) ainsi scellé est conditionné sous une atmosphère modifiée adaptée à protéger davantage le réceptacle anatomique (13) et, notamment, sa paroi interne, contre une contamination microbienne.

Selon un deuxième de ces aspects, comme illustré sur les figures 7-8, l'invention concerne un kit (2) de prélèvement urinaire pour homme comprenant le collecteur urinaire (1) déjà décrit ci-dessus. Selon l'invention, le collecteur urinaire (1) est stérile. Dans ces conditions, le kit (2) de prélèvement urinaire comprend un emballage étanche (200) adapté à envelopper le collecteur urinaire (1). L'emballage étanche (200) est conditionné sous une atmosphère modifiée adaptée à protéger le collecteur urinaire (1) contre une contamination microbienne.

De préférence, l'atmosphère modifiée comporte au moins un gaz inerte. De préférence, le kit (2) de prélèvement urinaire comporte un bouchon (50) stérile adapté à obturer le récipient (12).

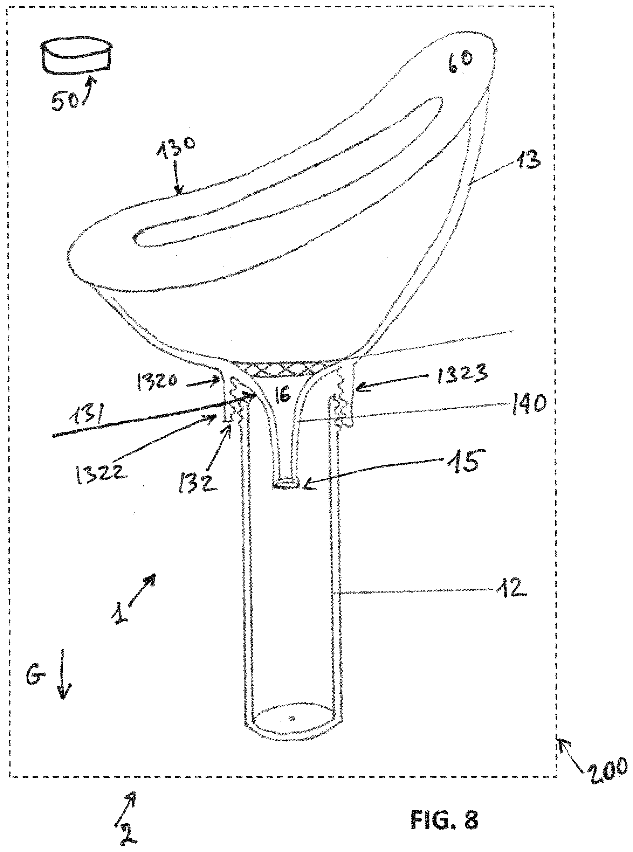

Le bord extérieur (130) du collecteur urinaire (1) peut comporter un gainage (par exemple, souple) référencé 60 sur les figures 7 et 8 adapté à épouser le corps humain de manière à éviter une coupure de la peau humaine.

Le kit (2) de prélèvement urinaire peut comporter collecteur urinaire (1) dans un état assemblé (le récipient 12 étant connecté au réceptacle anatomique (13)), comme illustré sur la figure 8, ou dans un état désassemblé (le récipient 12 étant déconnecté du réceptacle anatomique (13), comme illustré sur la figure 7.

Lors du prélèvement urinaire, l'homme (être humain du sexe masculin ou être humain du sexe féminin) enlève l'emballage étanche (200), ensuite approche le collecteur urinaire (1) dans l'état assemblé à son corps de manière à ce que le bord extérieur (130) du réceptacle anatomique (13) coopéré avec son corps au niveau du méat urétral, éjecte l'urine qui est stocker dans le récipient (12). Ensuite, le récipient (12) est désassemblé du réceptacle anatomique (13), et le bouchon (50) ferme le récipient (12). Puis, le récipient (12) bouché par le bouchon (50) est rendu au laboratoire pour analyses médicales.

## Revendications

1. Collecteur urinaire (1) pour homme comportant un récipient (12) ouvert vers l'extérieur et présentant un volume intérieur, le collecteur urinaire (1) comportant en outre un réceptacle (13) présentant un bord extérieur (130) destiné à être orienté vers un corps humain, ce bord extérieur (130) étant apte à coopérer avec le corps humain au niveau du méat urétral, le réceptacle (13) comprenant un orifice de sortie (131) disposé à l'opposé du bord extérieur (130), le récipient (12) étant adapté à être connecté de manière amovible au réceptacle (13), le récipient (12) étant en outre apte à coopérer avec l'orifice de sortie (131) du réceptacle (13), le réceptacle (13) étant adapté à capturer une urine éjectée du méat urétral lors de la miction et à canaliser cette urine, sous l'effet de la pesanteur (G), via l'orifice de sortie (131), vers le récipient (12), le volume intérieur du récipient (12) étant adapté à stocker l'urine issue du réceptacle (13), **caractérisé en ce que** le collecteur urinaire (1) est stérile, **en ce que** le réceptacle (13) est anatomique, et **en ce que** le récipient (12) comporte un appendice tubulaire ondulé flexible (120) apte à être déplié vers l'extérieur hors réceptacle anatomique (13) de manière à augmenter le volume intérieur dudit récipient (12).

2. Collecteur urinaire (1) selon la revendication 1, **caractérisé en ce qu'**au moins le réceptacle anatomique (13) est fabriqué en carton.

3. Collecteur urinaire (1) selon la revendication 2, **caractérisé en ce que** l'appendice tubulaire ondulé flexible (120) forme une bouche d'accès au volume intérieur du récipient (12), la bouche d'accès étant apte à coopérer avec l'orifice de sortie (131) du réceptacle anatomique (13).

4. Collecteur urinaire (1) selon l'une quelconque des revendications 1 à 3, **caractérisé en ce que** le réceptacle anatomique (13) est scellé au niveau du bord extérieur (130) et de l'orifice de sortie (131) par une pellicule étanche.

5. Collecteur urinaire (1) selon la revendication 4, **caractérisé en ce que** la pellicule étanche est en papier.

6. Collecteur urinaire (1) selon la revendication 4, **caractérisé en ce que** la pellicule étanche est en film de polymère.

7. Collecteur urinaire (1) selon l'une quelconque des revendications 4 à 6, **caractérisé en ce que** le réceptacle anatomique (13) scellé est conditionné sous une atmosphère modifiée adaptée à protéger sa paroi interne stérile contre une contamination microbienne.

8. Collecteur urinaire (1) selon l'une quelconque des revendications 1 à 7, **caractérisé en ce que** le réceptacle anatomique (13) comporte un dispositif de connexion (132) adapté à connecter de manière amovible le réceptacle anatomique (13) au récipient (12), **en ce que** ce dispositif de connexion (132) comporte une première extrémité (1320) liée solidairement au réceptacle anatomique (13) et une deuxième extrémité libre (1322), opposée à la première extrémité (1320), et **en ce que** le récipient (12) est adapté à être connecté à la deuxième extrémité libre (1322) à l'aide d'un lien amovible.

9. Collecteur urinaire (1) selon la revendication 8, **caractérisé en ce que** le lien amovible est de nature magnétique.

10. Collecteur urinaire (1) selon l'une quelconque des revendications 1 à 9, **caractérisé en ce que** le réceptacle anatomique (13) comporte un bec verseur disposé sur le bord extérieur (130), le bec verseur étant adapté à faciliter une évacuation d'un excédent de l'urine par-dessus bord du réceptacle anatomique (13).

11. Collecteur urinaire (1) selon la revendication 10, **caractérisé en ce que** le réceptacle anatomique (13) est fabriqué en matériau pliable, et **en ce que** le bec verseur est formé par pliage du réceptacle anatomique (13).

12. Collecteur urinaire (1) selon l'une quelconque des revendications 1 à 11, **caractérisé en ce qu'**il comporte un filtre d'urine disposé en amont du récipient (12).

13. Kit (2) de prélèvement urinaire pour homme comprenant un collecteur urinaire (1) selon l'une quelconque des revendications 1 à 12, ce kit (2) de prélèvement urinaire comprenant un emballage étanche (200) adapté à envelopper le collecteur urinaire (1), cet emballage étanche (200) étant conditionné sous une atmosphère modifiée adaptée à protéger le collecteur urinaire (1) contre une contamination microbienne.

14. Kit (2) de prélèvement urinaire selon la revendication 13, **caractérisé en ce qu'**il comporte un bouchon (50) stérile distinct du récipient (12), ce bouchon (50) stérile étant adapté à obturer le récipient (12) du collecteur urinaire (1).

15. Kit (2) de prélèvement urinaire selon la revendication 13 ou 14, **caractérisé en ce qu'**il comporte le collecteur urinaire (1) dans un état désassemblé, le récipient (12) étant déconnecté du réceptacle anatomique (13).

## Patentansprüche

1. Urinsammelbehälter (1) für Männer, aufweisend ein Behältnis (12), das nach außen offen ist und ein Innenvolumen vorweist, der Urinsammelbehälter (1) ferner aufweisend einen Behälter (13), der einen Außenrand (130) vorweist, der dazu bestimmt ist, einem menschlichen Körper zugewandt zu sein, wobei der Außenrand (130) geeignet ist, mit dem menschlichen Körper auf Höhe des Meatus urethrae zusammenzuwirken, der Behälter (13) umfassend eine Austrittsöffnung (131), die gegenüber des Außenrands (130) angeordnet ist, wobei das Behältnis (12) angepasst ist, um auf lösbare Weise mit dem Behälter (13) verbunden zu werden, wobei das Behältnis (12) ferner geeignet ist, mit der Austrittsöffnung (131) des Behälters (13) zusammenzuwirken, wobei der Behälter (13) angepasst ist, um Urin, der während der Miktion aus dem Meatus urethrae ausgestoßen wird, aufzufangen und diesen Urin unter der Wirkung der Schwerkraft (G) über die Austrittsöffnung (131) in das Behältnis (12) zu kanalisieren, wobei das Innenvolumen des Behältnisses (12) angepasst ist, um aus dem Behälter (13) stammenden Urin aufzubewahren, **dadurch gekennzeichnet, dass** der Urinsammelbehälter (1) steril ist, **dass** der Behälter (13) anatomisch ist und **dass** das Behältnis (12) einen flexiblen, gewellten, rohrförmigen Fortsatz (120) aufweist, der geeignet ist, aus dem anatomischen Behälter (13) heraus nach außen gefaltet zu werden, um das Innenvolumen des Behältnisses (12) zu vergrößern.

2. Urinsammelbehälter (1) nach Anspruch 1, **dadurch gekennzeichnet, dass** mindestens der anatomische Behälter (13) aus Pappe hergestellt ist.

3. Urinsammelbehälter (1) nach Anspruch 2, **dadurch gekennzeichnet, dass** der flexible, gewellte, rohrförmige Fortsatz (120) eine Zugangsmündung zu dem Innenvolumen des Behältnisses (12) bildet, wobei die Zugangsöffnung geeignet ist, mit der Austrittsöffnung (131) des anatomischen Behälters (13) zusammenzuwirken.

4. Urinsammelbehälter (1) nach einem der Ansprüche 1 bis 3, **dadurch gekennzeichnet, dass** der anatomische Behälter (13) auf Höhe des Außenrands (130) und der Austrittsöffnung (131) mit einer undurchlässigen Folie versiegelt ist.

5. Urinsammelbehälter (1) nach Anspruch 4, **dadurch gekennzeichnet, dass** die undurchlässige Folie aus Papier besteht.

6. Urinsammelbehälter (1) nach Anspruch 4, **dadurch gekennzeichnet, dass** die undurchlässige Folie aus einem Polymerfilm besteht.

7. Urinsammelbehälter (1) nach einem der Ansprüche 4 bis 6, **dadurch gekennzeichnet, dass** der versiegelte anatomische Behälter (13) unter einer modifizierten Atmosphäre verpackt ist, die angepasst ist, um seine sterile Innenwand vor einer mikrobiellen Kontamination zu schützen.

8. Urinsammelbehälter (1) nach einem der Ansprüche 1 bis 7, **dadurch gekennzeichnet, dass** der anatomische Behälter (13) eine Verbindungsvorrichtung (132) aufweist, die angepasst ist, um den anatomischen Behälter (13) auf lösbare Weise mit dem Behältnis (12) zu verbinden, **dass** diese Verbindungsvorrichtung (132) ein erstes Ende (1320), das fest mit dem anatomischen Behälter (13) verknüpft ist, und ein zweites freies Ende (1322), das dem ersten Ende (1320) gegenüberliegt, aufweist und **dass** das Behältnis (12) angepasst ist, um mittels einer lösbaren Verknüpfung mit dem zweiten freien Ende (1322) verbunden zu werden.

9. Urinsammelbehälter (1) nach Anspruch 8, **dadurch gekennzeichnet, dass** die lösbare Verknüpfung magnetischer Natur ist.

10. Urinsammelbehälter (1) nach einem der Ansprüche 1 bis 9, **dadurch gekennzeichnet, dass** der anatomische Behälter (13) einen an dem Außenrand (130) angeordneten Ausgießer aufweist, wobei der Ausgießer angepasst ist, um eine Entleerung von überschüssigem Urin über den Rand des anatomischen Behälters (13) zu erleichtern.

11. Urinsammelbehälter (1) nach Anspruch 10, **dadurch gekennzeichnet, dass** der anatomische Behälter (13) aus einem faltbaren Material hergestellt ist und **dass** der Ausgießer durch Falten des anatomischen Behälters (13) gebildet wird.

12. Urinsammelbehälter (1) nach einem der Ansprüche 1 bis 11, **dadurch gekennzeichnet, dass** er einen Urinfilter aufweist, der stromaufwärtig des Behältnisses (12) angeordnet ist.

13. Urin-Entnahmekit (2) für Männer, umfassend einen Urinsammelbehälter (1) nach einem der Ansprüche 1 bis 12, dieses Urin-Entnahmekit (2) umfassend eine dichte Verpackung (200), die angepasst ist, um den Urinsammelbehälter (1) zu umhüllen, wobei diese undurchlässige Verpackung (200) unter einer modifizierten Atmosphäre verpackt ist, die angepasst ist, um den Urinsammelbehälter (1) vor einer mikrobiellen Kontamination zu schützen.

14. Urin-Entnahmekit (2) nach Anspruch 13, **dadurch gekennzeichnet, dass** es einen sterilen Stopfen (50) aufweist, der sich von dem Behältnis (12) unterscheidet, wobei dieser sterile Stopfen (50) angepasst ist, um das Behältnis (12) des Urinsammelbehälters (1) zu verschließen.

15. Urin-Entnahmekit (2) nach Anspruch 13 oder 14, **dadurch gekennzeichnet, dass** es den Urinsammelbehälter (1) in einem zerlegten Zustand aufweist, wobei das Behältnis (12) von dem anatomischen Behälter (13) getrennt ist.

## Claims

1. Urine collector (1) for humans comprising a container (12) which is outwardly open and having an interior volume, the urine collector (1) further comprising a receptacle (13) having an outer edge (130) which is intended to be oriented toward a human body, this outer edge (130) being capable of engaging with the human body at the urethral meatus, the receptacle (13) comprising an outlet opening (131) arranged opposite the outer edge (130), the container (12) being suitable for being releasably connected to the receptacle (13), the container (12) being further capable of engaging with the outlet opening (131) of the receptacle (13), the receptacle (13) being suitable for capturing urine ejected from the urethral meatus during urination and for channeling this urine, under the effect of gravity (G), via the outlet opening (131), to the container (12), the interior volume of the container (12) being suitable for storing the urine from the receptacle (13), **characterized in that** the urine collector (1) is sterile, **in that** the receptacle (13) is anatomical, and **in that** the container (12) comprises a flexible corrugated tubular appendage (120) which is capable of being unfolded outwards out of the anatomical receptacle (13) so as to increase the interior volume of said container (12).

2. Urine collector (1) according to claim 1, **characterized in that** at least the anatomical receptacle (13) is made of cardboard.

3. Urine collector (1) according to claim 2, **characterized in that** the flexible corrugated tubular appendage (120) forms a mouth for accessing the interior volume of the container (12), the access mouth being capable of engaging with the outlet opening (131) of the anatomical receptacle (13).

4. Urine collector (1) according to any one of claims 1 to 3, **characterized in that** the anatomical receptacle (13) is sealed at the outer edge (130) and the outlet opening (131) by a sealed film.

5. Urine collector (1) according to claim 4, **characterized in that** the sealed film is made of paper.

6. Urine collector (1) according to claim 4, **characterized in that** the sealed film is made of polymer film.

7. Urine collector (1) according to any one of claims 4 to 6, **characterized in that** the sealed anatomical receptacle (13) is packaged under a modified atmosphere suitable for protecting its sterile internal wall against microbial contamination.

8. Urine collector (1) according to any of claims 1 to 7, **characterized in that** the anatomical receptacle (13) comprises a connection device (132) which is suitable for releasably connecting the anatomical receptacle (13) to the container (12), **in that** the connection device (132) comprises a first end (1320) rigidly connected to the anatomical receptacle (13) and a second free end (1322), opposite the first end (1320), and **in that** the container (12) is suitable for being connected to the second free end (1322) using a releasable link.

9. Urine collector (1) according to claim 8, **characterized in that** the releasable link is magnetic.

10. Urine collector (1) according to any one of claims 1 to 9, **characterized in that** the anatomical receptacle (13) comprises a pouring spout arranged on the outer edge (130), the pouring spout being suitable for facilitating an excess of the urine being discharged over the edge of the anatomical receptacle (13).

11. Urine collector (1) according to claim 10, **characterized in that** the anatomical receptacle (13) is made of a foldable material, and **in that** the pouring spout is formed by folding the anatomical receptacle (13).

12. Urine collector (1) according to any one of claims 1 to 11, **characterized in that** it comprises a urine filter arranged upstream of the container (12).

13. Urine collection kit (2) for humans, comprising a urine collector (1) according to any one of claims 1 to 12, this urine collection kit (2) comprising a sealed packaging (200) which is suitable for housing the urine collector (1), this sealed packaging (200) being packaged under a modified atmosphere suitable for protecting the urine collector (1) against microbial contamination.

14. Urine collection kit (2) according to claim 13, **characterized in that** it comprises a sterile plug (50) which is separate from the container (12), this sterile plug (50) being suitable for closing the container (12) of the urine collector (1).

15. Urine collection kit (2) according to claim 13 or claim 14, **characterized in that** it comprises the urine collector (1) in a disassembled state, the container (12) being disconnected from the anatomical receptacle (13).
